(19) Europäisches Patentamt
European Patent Office
Office européen des brevets

(11) **EP 4 497 430 A1**

(12) **EUROPEAN PATENT APPLICATION**
published in accordance with Art. 153(4) EPC

(43) Date of publication:
**29.01.2025 Bulletin 2025/05**

(21) Application number: 23774058.4

(22) Date of filing: **15.03.2023**

(51) International Patent Classification (IPC):
**A61K 8/97** (2017.01)    **A61Q 19/08** (2006.01)

(52) Cooperative Patent Classification (CPC):
**A61K 8/97; A61Q 19/08**

(86) International application number:
**PCT/ES2023/070157**

(87) International publication number:
**WO 2023/180600 (28.09.2023 Gazette 2023/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.03.2022 ES 202230255**

(71) Applicant: **Monteloeder, S.L.
03203 Elche (Alicante) (ES)**

(72) Inventor: **CATURLA CERNUDA, Nuria
03203 Elche / Alicanteǫ(ES)**

(74) Representative: **Martin Alvarez, Juan Enrique
Ibidem Abogados Estrategas SLP
B54357488
Juan de la Cierva, 43, Planta 2 Local 1-1
03203 Elche (Alicante) (ES)**

(54) **COMPOSITION OF PLANT EXTRACTS FOR REDUCING THE EFFECTS OF AGING BY PREVENTING OR DELAYING CELLULAR SENESCENCE**

(57)    Ageing is characterised by a progressive loss of physical and functional integrity, which progressively affects the body's health. A peculiar feature of ageing is the increase in the number of senescent cells, which have entered a state of irreversible arrest of cell cycles after exceeding an inherent or environmental stress level. Although cellular senescence is an essential process for several physiological processes, it plays a detrimental role in a large number of age-related pathologies. A first aspect of the invention provides a composition of plant extracts to mitigate the effects of ageing comprising an asiaticoside content of at least 6.0 % by weight, prefer-ably from a centella asiatica extract, a verbascoside content of at least 2.5 % by weight, preferably from a cistanche extract, a hesperidin content of at least 12.5 % by weight, preferably from a sweet orange extract and a punicalagin content of at least 3.0 % by weight, preferably from a pomegranate extract. A composition such as that described above prevents the negative effects of ageing by reducing the number of cells reaching the senescent state, due to the ability of said formulation to prevent or reduce telomere shortening during cell division. This composition is particularly indicated for preventing the effects of ageing on the skin.

EP 4 497 430 A1

**Description**

**TECHNICAL FIELD OF THE INVENTION**

The present invention is included within the technical field of products for delaying or reducing the effects of ageing on cellular tissues, particularly the skin, but also on internal organs and tissues, by means of an alternative treatment to the usual ones, based on delaying and/or preventing cellular senescence.

**BACKGROUND OF THE INVENTION**

Ageing is a physiological decline in structural homeostasis, as well as in functional integrity, which progressively affects the health of the organism (Shulin et al, 2020). A peculiar feature of ageing is the increase in the number of senescent cells, which have entered a state of irreversible arrest of cell cycles after exceeding an inherent or environmental stress level. Senescence is a consequence of telomere shortening after repeated cell divisions and although cellular senescence is an essential process for several physiological processes, it plays a detrimental role in a large number of age-related pathologies. However, current anti-ageing products for the skin are limited to correcting the visible effects of ageing, such as wrinkles, sagging, lack of brightness, etc., while other effects of ageing that are not as visible, i.e. pathological effects, such as decreased protection against pathogens, increased susceptibility to irritation, decreased healing ability and increased susceptibility to cancer, remain untreated.

References

- Shuling Song,Tamara Tchkonia,Jing Jiang,James L. Kirkland,Yu Sun; "Targeting Senescent Cells for a Healthier Aging: Challenges and Opportunities"; published in Advanced Science, Dec. 2020, Vol 7, Issue 23 (Wiley).

**BRIEF DESCRIPTION OF THE INVENTION**

A first aspect of the invention provides a composition of plant extracts to mitigate the effects of ageing comprising an asiaticoside content of at least 6% by weight, a verbascoside content of at least 2.5% by weight, a hesperidin content of at least 12.5% by weight, and a punicalagin content of at least 3% by weight.
As will be illustrated in the accompanying examples, a composition such as that described above prevents the negative effects of ageing, both visible and pathological, by reducing the number of cells reaching the senescent state, due to the ability of said formulation to prevent or reduce telomere shortening during cell division and under suitable conditions, to prolong telomere length by stimulating telomerase enzyme activity. By preventing or delaying telomere shortening, the skin is not only prettier, but also healthier, because when epithelial stem cells have very short telomeres they do not leave their epithelial niches, nor regenerate the skin and hair properly, which produces premature ageing. This composition also improves the state of health of all tissues and organs, both external and internal.
Telomeres are critical structures at the end of the chromosomes of eukaryotic organisms composed of copies of guanine-rich repeats. In mammals, the TTAGGG telomeric repeat is repeated thousands of times (Moyzis et al., 1988). Telomeres protect the ends of chromosomes from degradation and from being recognised as breaks in the DNA double helix. Without telomeres, chromosomes would fuse and genetic instability would occur (O'Sullivan and Karlseder, 2010). In humans, the average telomere length decreases from 11 kilobases at birth to less than 4 kilobases in old age (Arai et al. 2015). During chromosome replication, enzymes that duplicate DNA cannot continue their duplication until the end of the chromosomes, so that the end of the chromosomes is shortened at each duplication. Telomeres form the ends or tips of human chromosomes and, therefore, shorten in each round of cell division. This mechanism limits human cell proliferation to a finite number of cell divisions by inducing replicative senescence, differentiation or apoptosis (Miyata et al. 2004; Smeets et al., 2011).
Telomere length, therefore, decreases over time and can predict life expectancy. Telomere shortening has negative health effects and has been associated with cellular senescence, and many other health problems including ageing and cancer. An accurate and consistent quantification of telomere length is important in many aspects of cell biology, such as chromosomal instability, DNA repair, senescence, apoptosis, cell dysfunctions and oncogenesis.
In this regard, the skin is a self-regenerating tissue that goes through an extensive proliferation throughout the life of an organism. Telomeres in epithelial cells are particularly susceptible to accelerated telomere shortening due to proliferation and DNA damaging agents, such as solar radiation, environmental pollution, or radical oxygen species (Buckingham and Klingelhuts, 2011; D'Errico et al., 2007).
Preferably, the asiaticoside content is provided as a centella asiatica extract. Preferably, the composition contains about 36% by weight of centella asiatica extract.

Centella asiatica is a source rich in asiaticosides and, therefore, is a suitable plant to provide the required asiaticoside content, without having to include a large amount of inert or inactive compounds in the composition.

Preferably, the verbascoside content is provided as a cistanche extract. Preferably the composition contains about 32% by weight of Cistanche extract. The cistanche extract may be of the following Cistanche varieties: *Cistanche tubulosa, Cistanche salsa* and/or *Cistanche deserticola.*

Cistanche is a rich source of verbascosides and, therefore, is a suitable plant to provide the required verbascoside content, without having to include a large amount of inert or inactive compounds in the composition.

Optionally, the verbascoside content may also be provided with extracts from other plants such as *Lippia citriodora, Budeja sp., Rehmania glutinosa, Plantago spp.*

Preferably, the hesperidin content is provided as a citrus extract, i.e. *Citrus sinensis, Citrus amara ssp. sinensis variety dulcis,* among many others. Preferably, the composition contains about 23% sweet orange extract.

Citrus fruits constitute a source rich in hesperidin and are, therefore, suitable plants to provide the required hesperidin content, without having to include a large amount of inert or inactive compounds in the composition.

Preferably, the punicalagin content is provided as a pomegranate extract (*Punica granatum*). Preferably, the composition contains about 9% pomegranate extract.

Pomegranate is a source rich in punicalagins and is, therefore, a suitable plant to provide the required punicalagin content, without having to include a large amount of inert or inactive compounds in the composition.

Administration of the compositions of the present invention is selected from formulations containing it intended for oral, topical, transdermal or parenteral administration.

Preferably, the compositions of the present invention may be administered orally containing one or more physiologically compatible carriers or excipients, in solid or liquid form. These formulations may contain conventional components such as physiologically acceptable binding agents, fillers, lubricants and wetting agents. The formulations may take any appropriate form, such as tablets, pills, capsules, lozenges, oily or aqueous solutions, suspensions, emulsions or in dry powder form suitable for reconstitution with water or other suitable liquid medium prior to use, for immediate or controlled release.

Liquid oral forms for administration may also contain certain additives such as sweeteners, flavourings, preservatives and emulsifying agents. Non-aqueous liquid compositions for oral administration may also be formulated, for example, containing edible oils. Such liquid compositions can be conveniently encapsulated in, for example, gelatin capsules in a unit dosage amount.

Preferably, the composition is administered in doses of 100 mg to 1 g daily, and more preferably between 100 and 300 mg daily.

The compositions of the present invention may be administered parenterally in combination with conventional injectable liquid carriers, such as water or suitable alcohols. Conventional pharmaceutical adjuvants for injection, such as stabilising agents, solubilising agents and buffers may be included in such injectable formulations. The composition of the present invention may be administered intramuscularly, intraperitoneally or intravenously.

Likewise, the compositions of the present invention can be administered topically, topical application formulations containing one or more physiologically compatible skin carriers or excipients, in liquid, aerosol, cream, etc.

References

- Buckingham EM, Klingelhutz AJ. The role of telomeres in the ageing of human skin. Exp Dermatol. 2011 Apr;20(4):297-302.
- D'Errico M, Lemma T, Calcagnile A, Proietti De Santis L, Dogliotti E. Cell type and DNA damage specific response of human skin cells to environmental agents. Mutat Res. 2007 Jan 3;614(1-2):37-47.
- Miyata Y, Okada K, Fujimoto A, Hata K, Kagami H, Tomita Y, Ueda M. The effect of long-term cultivation on telomere length and morphology of cultured epidermis. J Dermatol Sci. 2004 May;34(3):221-30.
- Moyzis RK, Buckingham JM, Cram LS, Dani M, Deaven LL, Jones MD, Meyne J, Ratliff RL, Wu JR. A highly conserved repetitive DNA sequence, (TTAGGG)n, present at the telomeres of human chromosomes. Proc Natl Acad Sci U S A. 1988 Sep; 85(18):6622-6.
- O'Sullivan RJ, Karlseder J. Telomeres: protecting chromosomes against genome instability. Nat Rev Mol Cell Biol. 2010 Mar; 11(3):171-81.
- Smeets SJ, van der Plas M, Schaaij-Visser TB, van Veen EA, van Meerloo J, Braakhuis BJ, Steenbergen RD, Brakenhoff RH. Immortalization of oral keratinocytes by functional inactivation of the p53 and pRb pathways. Int J Cancer. 2011 Apr 1;128(7):1596-605.

## BRIEF DESCRIPTION OF THE FIGURES

Figure 1 illustrates the effect of senescent cells on epithelial tissues.

Figure 2 shows an illustrative graph of the effect of a composition according to the present invention on cell proliferation of normal human dermal fibroblasts (MTT assay).

Figure 3 shows an illustrative graph of the antioxidant effect of a composition according to the present invention on normal human dermal fibroblast cultures.

Figure 4 shows an illustrative graph of the effect of a composition according to the present invention on telomerase activity in normal human dermal fibroblasts.

Figure 5 shows an illustrative graph of the effect of a composition according to the present invention on telomere length in normal human dermal fibroblasts.

Figure 6 shows an illustrative graph of the effect of a composition according to the present invention on telomere shortening in normal human dermal fibroblasts relative to the number of cell divisions.

Figure 7 shows an illustrative graph of the effect of a composition according to the present invention on telomere shortening in normal human dermal fibroblasts relative to the number of cell divisions per base pair.

Figure 8 shows an illustrative graph of the effect of a composition according to the present invention on melanin production in normal human epidermal melanocytes exposed to ultraviolet radiation.

Figure 9 shows an illustrative graph of the effect of a composition according to the present invention on cell viability in normal human epidermal melanocytes exposed to ultraviolet radiation.

## DETAILED DESCRIPTION OF THE INVENTION AND EXAMPLES

The following description details with examples how, surprisingly, composition A according to the present invention can mitigate the effects of ageing by preventing or reducing the rate at which epithelial cells enter senescence.

Composition A comprises 36.4 % by weight of centella asiatica extract, 31.8 % by weight of cistanche extract, 22.7 % by weight of sweet orange extract and 9.1 % by weight of pomegranate extract.

Centella asiatica extract contains about 20% asiaticosides quantified by High Performance Liquid Chromatography (HPLC)

The cistanche extract contains about 10% verbascoside quantified by HPLC.

The sweet orange extract contains about 60% hesperidin as quantified by HPLC.

The pomegranate extract contains about 40% punicalagins quantified by HPLC.

### Epithelial ageing

Skin ageing is a process wherein morphological and functional changes progressively occur in the skin. There is a decline in biological functions and in the ability to adapt to metabolic stress that ultimately results in the appearance of wrinkles, sagging and changes in pigmentation, among others.

The determining factors of skin ageing can be internal and external. Intrinsic ageing is caused by genetic determinants. While extrinsic ageing depends on exposure to various environmental factors (UV rays, pollution, etc.) and lifestyle (smoking, food, sleep and stress).

On a cellular level, fibroblasts, the most important cell type of the dermis, are essential for producing the collagens and other proteins that form the extracellular matrix, and that allow the skin to maintain its function properly. As we age, the number of fibroblasts progressively decreases as they lose their proliferative capacity and their function progressively deteriorates, for example, reducing the expression of genes encoding collagen isoforms.

Furthermore, the number of senescent fibroblasts increases with age, due, among other causes, to deterioration of senescent cell elimination systems.

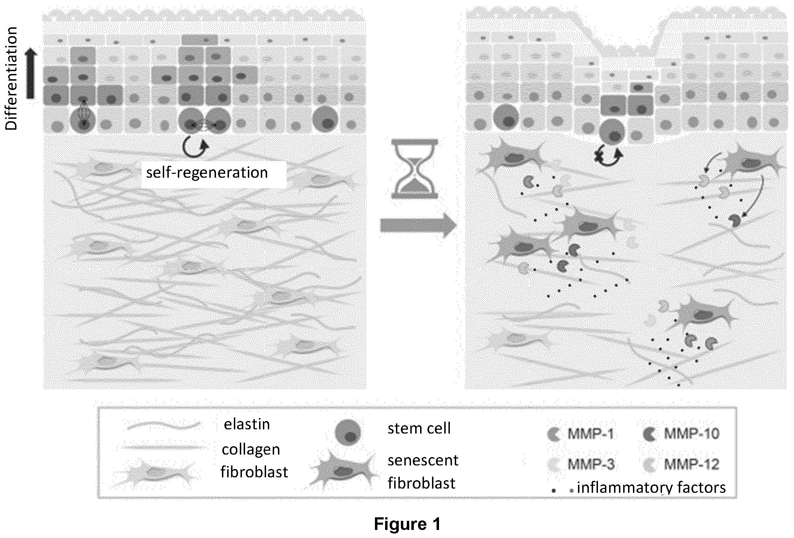

Senescent cells are harmful because they develop into a senescence-associated secretory phenotype (SASP), which is considered one of the prime causes of ageing. Human fibroblasts that have adopted SASP secrete pro-inflammatory cytokines and matrix metalloproteinases (MMPs) and release reactive oxygen species. All this leads to degradation of the extracellular matrix and consequently skin ageing (see Figure 1 (Freitas *et al.,* 2007)).

Additionally, reactive oxygen species (ROS) play a critical role in both intrinsic and extrinsic ageing. With increasing age, the balance between ROS and antioxidant agents is lost in favour of the former, resulting in activation of multiple MAP kinase pathways, which induce

an increase in matrix metalloproteinase (MMP) synthesis, responsible for the collagen degradation of human skin. This imbalance between the enzymes that

remodel and repair the dermal matrix favours the loss of connective tissue and atrophy of the skin. This is associated with the loss of immune function.

For this reason, Examples 1 and 2 evaluate the anti-ageing effects of composition A according to the present invention after *in vitro* treatment of normal human dermal fibroblasts (NHDF) subjected to culture conditions simulating the ageing process (cell senescence), by quantifying cell viability with the MTT (cell proliferation) assay and ROS after exposure to a medium containing hydrogen peroxide ($H_2O_2$).

References

Sandra Freitas-Rodríguez, Alicia R. Folgueras, Carlos López-Otín, The role of matrix metalloproteinases in aging: Tissue remodeling and beyond, Biochimica et Biophysica Acta (BBA) - Molecular Cell Research,(2007) 1864, Issue 11, Part A, 2015-2025.

**EXAMPLE 1. Formulation A according to the present invention prevents the loss of fibroblast proliferation potential in ageing medium by protecting them from cellular senescence.**

Methodology

For the cell proliferation assay, normal human dermal fibroblasts (NHDF) were exposed for 3 hours to conditioned ageing medium containing $H_2O_2$ (500$\mu$M). After the incubation period, the medium was replaced with fresh medium containing composition A according to the invention at different concentrations for 96 hours. Epidermal growth factor (EGF, 20 ng/mL) was included as positive control and NHDF without pretreatment in $H_2O_2$ as non-aged control (Control). Cell viability was quantified using the MTT assay.

Results

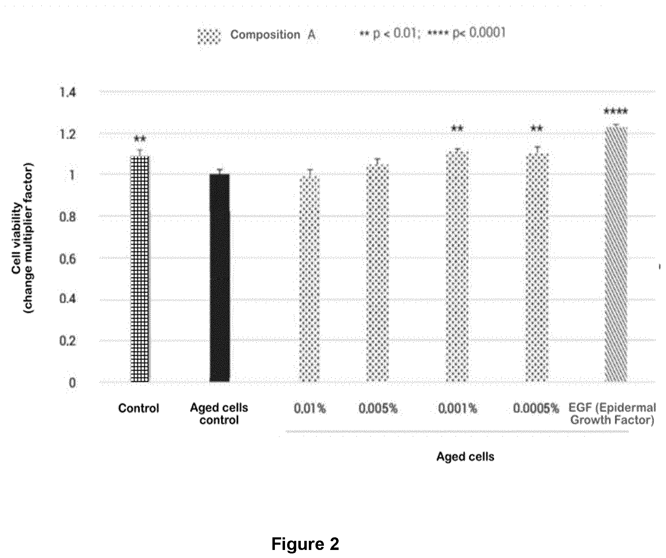

The results obtained can be observed in the graph of Figure 2.

$H_2O_2$ pretreated fibroblasts (aged control) exhibited markedly reduced cell viability (-9.2%) compared to that of the non-aged control. Cells supplemented with composition A according to the invention regained their proliferation capacity and a significant increase in cell viability was observed at 0.001 % by weight (+11.3%) and at 0.0005% by weight (+10.8%) compared to the aged control, raising the growth capacity to levels similar to that of the non-aged fibroblasts, and somewhat lower than the level of the control cells incubated with the epidermal growth factor EGF (+22.9%).

This example demonstrates the potent efficacy of composition A according to the invention in counteracting the effects of cell ageing on the regenerative ability of tissues.

**EXAMPLE 2. Formulation A according to the present invention inhibits $H_2O_2$-induced oxidative stress, conditioned ageing medium, in human fibroblasts.**

Methodology

Normal human dermal fibroblasts (NHDF) were exposed for 3 hours to conditioned ageing medium containing $H_2O_2$ (500).mMAfter incubation, the medium was replaced with fresh medium containing composition A according to the invention at different concentrations for 24 hours.). After this period, the production of radical oxygen species (ROS) was measured by fluorometry. ROS generation measurements were calculated against cells untreated with composition A and aged with $H_2O_2$ (aged control).

Results

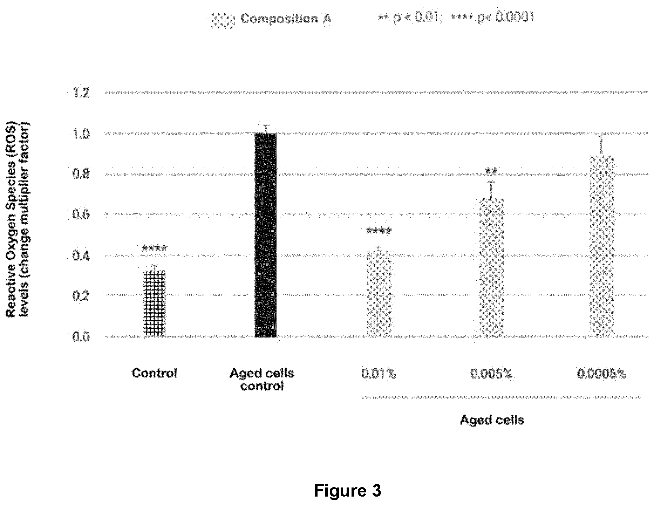

The results obtained can be observed in the graph of Figure 3.

The dose of senescence-producing $H_2O_2$ exhibited a significantly higher level of intracellular ROS in cells untreated with composition A ($\pm$ 60.4%), which is a characteristic of skin ageing. Whereas when the cells were treated with composition A for 24 hours, the ROS level decreased. Treatment with composition A at 0.005% and 0.01% by weight decreased ROS levels by 32% and 57.7%, respectively.

This example demonstrates the potent efficacy of composition A according to the invention in reducing the effects of cellular stress on the ability to produce oxidising free radicals.

**Telomeres and senescence**

Telomeres are dynamic DNA-protein complexes that protect the ends of chromosomes and promote chromosomal stability. When cells divide, telomeres are not replicated in their entirety which implies a shortening thereof in each replication (Chan and Blackburn, 2004). Once telomeres reach a critical length, the cells stop dividing and enter senescence (Victorelli and Passos, 2017). Hence, telomere length is considered a marker of cellular ageing. The telomere shortening process, as we age, is reduced by the enzyme telomerase, whose function is to add nucleotides to the end of the end of the DNA molecule (Oeseburg H, 2010).

Telomerase activity is found in certain cells (e.g. germ cells and stem cells), which divide continuously and must maintain telomeres above a certain length in order to perform their functions. By stimulating telomerase activity, it is possible to increase telomere length and consequently extend the number of cell divisions that can take place without incurring harmful chromosomal aberrations or telomere fusion (Sahin E, 2010). Although this does not make the cells immortal, it can extend their lifecycle. Furthermore, the amount of telomerase in our bodies decreases as we age (Eisenberg, 2011).

Thus, by activating telomerase you can:

- Tackle telomere shortening and cellular ageing
- Help the cells live longer and continue to function properly.
- Make old cells work like they did when they were young (by changing gene expression to a younger phenotype).

**EXAMPLE 3A. Composition A according to the present invention exhibits anti-ageing effects and protection effects against senescence by stimulating telomerase activity.**

Methodology

Determination of the relative telomerase activity by Q-TRAP

The DNA polymerase chain reaction (PCR)-based quantitative telomeric repeat amplification protocol is a sensitive and accurate PCR-based assay that allows measurement of telomerase activity.

Cell pellets were split using CHAPS division buffer ((3-cholamidopropyl)dimethylammonium)-1-propanesulfonate for protein extraction.

Samples were stored at 4°C and used within 24 hours. Protein quantification was performed for each sample using the Bio-Rad protein assay (Bio-Rad Kit 50000002). A minimum protein concentration of 0.3 mg/mL is required to perform the sample analysis. Telomerase protein extracts were incubated at 27°C for 30 minutes to allow telomerase from protein extracts to elongate telomeres by addition of TTAGGG repeat sequences. After the enzymatic reaction, telomerase extension products were amplified and quantified by real-time qPCR using SYBR green (a fluorescent cyanine pigment). PCR was initiated at 95°C for 10 minutes, followed by a 40 cycle amplification (95°C for 15 s, 60°C for 60 s) and a fusion curve step. Reactions were monitored and analysed with QuantStudio 5 (Applied Biosystems). Telomerase activity in the cell lines or samples was calculated based on the threshold cycle (Ct). All samples were run in triplicate.

The telomerase positive standard dilution series is plotted against telomerase protein concentration (r2>0.9) as a standard curve of Ct values. The standard curve is generated by plotting threshold cycles (Ct values) of standard HeLa cell line against the logarithm of 1000, 333, 111, 37.03, 12.34, 4.11, 1.37 and 0.45 ng protein (whole cell extract).

Telomerase activity was measured in human fibroblasts over a period of 24 and 72 hours.

Results

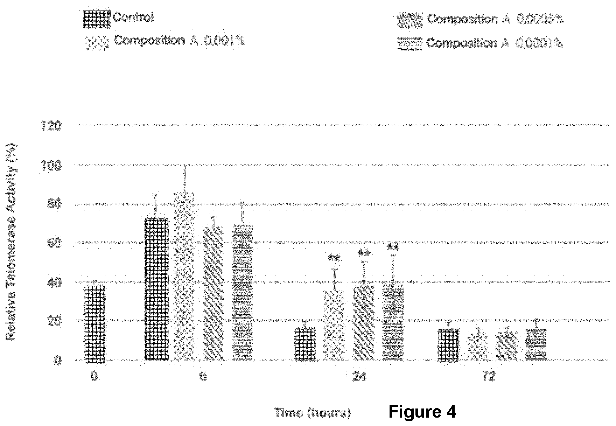

The results of the telomerase activity assay are shown in the graph of Figure 4.

After six hours of assay, the control and samples treated with composition A according to the present invention showed similar levels of telomerase activity. An insignificant increase in telomerase activity was only observed in the sample treated with composition A at 0.001 % by weight of concentration.

After 24 hours, increased telomerase activity was observed in all cell samples treated with composition A, compared to the untreated control group. These differences were identified as statistically significant (p<0.01%).

After 72 hours, the control and treated samples showed similar levels of telomerase activity.

References

- Chan SR, Blackburn EH. 2004. Telomeres and telomerase. Philos Trans R Soc Lond B Biol Sci. 359(1441):109-121. doi:10.1098/rstb.2003.1370
- Victorelli S, Passos JF. 2017. Telomeres and cell senescence - size matters not. EBioMedicine. 21:14-20. doi:10.1016/j.ebiom.2017.03.027.
- Eisenberg DTA. An evolutionary review of human telomere biology: the thrifty telomere hypothesis and notes on potential adaptive paternal effects. American Journal of Human Biology. 2011;23:149-167.
- Oeseburg H, et al. Telomere biology in healthy aging and disease. Pflügers Archiv - European Journal of Physiology. 2010;459:259-268
- Sahin E, DePinho RA. Linking functional decline of telomeres, mitochondria and stem cells during ageing. Nature. 2010;464:520-528.
- Hou M, Xu D, Bjorkholm M, Gruber A. 2001. Real-time quantitative telomeric repeat amplification protocol assay for the detection of telomerase activity. Clin Chem. 47(3):519-524.

**EXAMPLE 3B. Composition A according to the present invention exhibits anti-ageing effects and protective effects against senescence by preventing telomere shortening.**

To test whether composition A is capable of at least partially inhibiting telomere shortening, two different experiments have been performed with two different techniques and different conditions.

PROTOCOL 1

Methodology

For the telomere length experiment, NHDF fibroblasts were cultured and grown for 24-29 cell doublings, inducing ageing and telomere shortening due to cell passaging. Throughout this culture, the cells were treated or untreated (untreated control) with composition A, substituting the medium for fresh product every 3 days. After incubation, the cells were harvested and the gDNA was extracted and the length of their telomeres was quantified by RT-qPCR. The gDNA was also extracted from the cells at time 0. The number of cell doublings was calculated by cell counting.

To calculate telomere length, a reference human genomic DNA sample was included in the qPCR reaction, which had a known telomere length. To perform the analysis of the primary data, the Pfaffl method (Pfaffl, 2001) was used to calculate the ratio of gene expression to SCR (internal control). The mathematical expression of the relative gene expression in real time PCR is as follows:

$$\text{Proportion} = \frac{(E_{target})^{\Delta CP_{target}(control-samp)}}{(E_{reference})^{\Delta CP_{reference}(control-samp)}}$$

Results

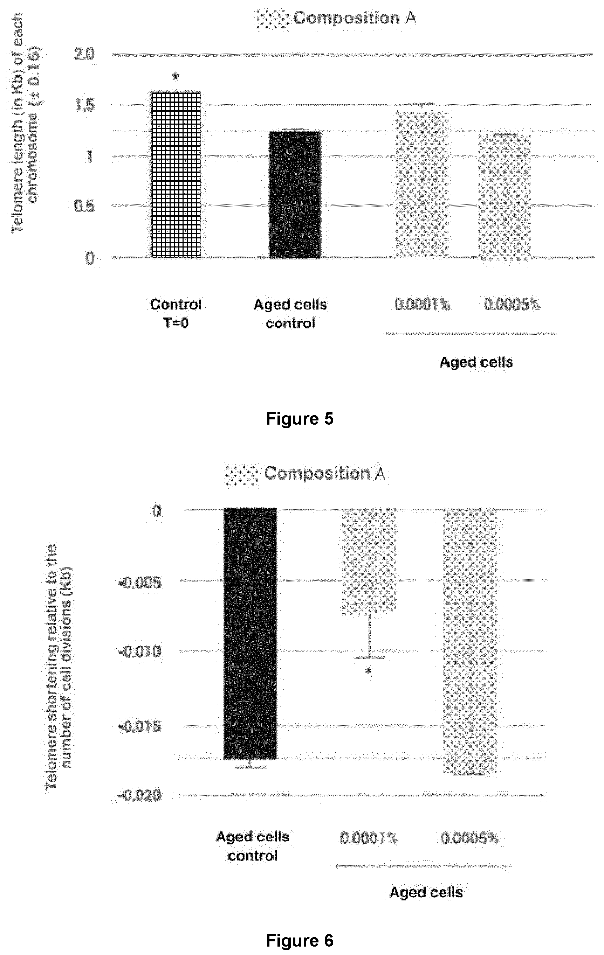

The results obtained can be observed in the graph of Figure 5.

Fibroblasts aged for 24-29 doublings without treatment showed significantly reduced telomere length, compared to the non-ageing control. However, treatment of fibroblasts with a concentration as low as 0.0001% by weight of composition A significantly increased telomere length compared to untreated aged cells.

To better reflect the telomere shortening resulting from cell division, the telomere shortening values were normalised with respect to the estimated number of cell divisions that occurred under the various conditions. When this correction is applied, the results (shown in Figure 6) show a statistically significant decrease in telomere shortening caused by Composition A at 0.0001 % by weight of concentration (p < 0.05). Under these conditions, telomeres shortened at a rate of 0.010 ± 0.003 kb/division slower than in untreated cells. This figure is 57.7% slower than the untreated control.

PROTOCOL 2

Methodology

For this experiment, normal human fibroblasts were cultured and grown for 8 weeks (14-17 cell doublings) together with composition A at three different concentrations, 0.001 %, 0.0005% and 0.0001 % by weight. The cells were cultured and tested under oxidative stress conditions by adding $H_2O_2$ (10 $\mu$M) as oxidative stress agent. Alterations in telomere lengths and telomere shortening rates (TSRs) were determined for each concentration of composition A and the results were compared.

The telomere length measurement was performed using the Telomere Analysis Technology (TAT[®]) owned by Life Length. TAT[®] measures telomere length using the previously described HT Q-FISH (high-throughput quantitative fluorescence in situ hybridization) high productivity technique (de Pedro et al, 2020). This method is based on a modified quantitative fluorescence in situ hibridization method for measuring individual chromosomes in interphase cells. Briefly, telomeres hybridise to a fluorescent peptide nucleic acid (PNA) that recognises three telomere repeats (the sequence: Alexa488-OO-CCCTAACCCTAACCCTAA, purchased from Panagene). Images of the nuclei and telomeres are captured by a high-content display system. The intensity of the telomere PNA fluorescent signal that hybridises to a given telomere is proportional to the length of said telomere. Fluorescence intensity is transformed into base pairs (bp) by a standard regression curve that is generated using control cell lines with known telomere length. TAT[®] not only measures telomere length in absolute base pairs, it also provides an assessment of telomere length distribution and the percentage of short telomeres, thus allowing a more complete analysis of each sample.

Three distinct variables were thus measured with the TAT[®] method: the median telomere length, the 20th percentile of the telomere length and the shortest telomere percentage of 3 Kbp which is perceived as the amount of critically short telomeres that increases the risk of cells entering senescence.

All samples were analysed in quintuplicate.

Normal human dermal fibroblasts were used to observe the effect of composition A on telomeres under conditions of oxidative stress (10 $\mu$g/ml $H_2O_2$) over a period of 6 and 8 weeks.

Results

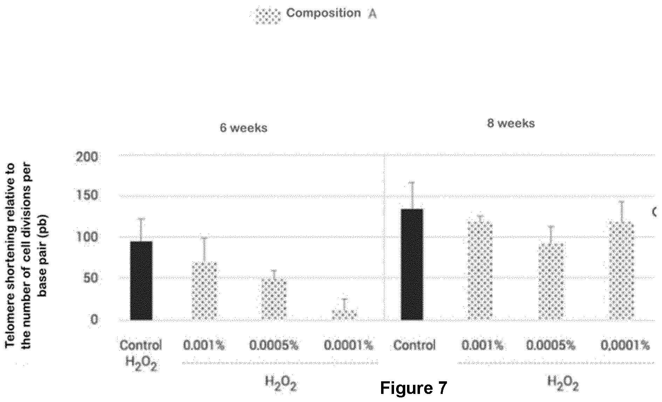

The results obtained can be observed in the graph of Figure 7.

The results show that untreated fibroblasts aged for 6 and 8 weeks under oxidative stress exhibit a shorter telomere length as well as a higher percentage of short telomeres compared to non-aged fibroblasts (time 0 control). In contrast, fibroblasts treated with composition A exhibited higher median telomere length and 20th percentile values, as well as a lower percentage of short telomeres (<3Kb), compared to the untreated control group, during both 6 and 8 weeks of treatment.

Furthermore, after normalising the data with the duplicate population, a slower rate of telomere shortening was observed in fibroblasts treated with composition A, suggesting a protective effect of telomeres under oxidative stress and ageing conditions.

These experiments demonstrate the potent efficacy of composition A according to the invention to delay cell progression to a state of senescence, by a reduction in the rate of telomere shortening.

Our skin ages because, individually, every cell in the body ages and enters a phase of senescence, depending on the chronology established by a biological cell clock: the telomeres.

Telomeres are repeating units of DNA that protect the ends of chromosomes during cell division. As the cells divide over time, the telomeres shorten and when the length of the telomeres is very short, the cells end their replication by division and enter senescence.

Senescent cells are found in greater numbers in older ages due to an age-related deterioration of senescent cell elimination systems, such as the immune system and autophagy-lysosomal cycle.

Senescent cells are harmful because they evolve into a senescence-associated secretion phenotype (SASP), which is suspected to be one of the major causes of ageing. SASP cells communicate with neighbouring cells using pro-inflammatory cytokines, which include catabolic modulators such as matrix metalloproteinases (MMPs) (see Figure 1, right), and release oxygenated radical species, resulting in degradation of the cell matrix in its surroundings.

**Environmental and ageing effects on skin pigmentation (UVA radiation)**

The skin pigmentation system is modified by the ageing process and irregular pigmentation is one of the greatest age-related changes.

Melanin secreted by melanocytes is the most important pigment of human skin at the base of the epidermis. Melanin is vital for the photoprotection of human skin from the sun's harmful rays. However, there are several epithelial hyperpigmentation disorders resulting from melanin overproduction and subsequent accumulation, such as freckles, age spots, melasma, and senile lentigo, which can be a distressing problem. This overproduction is triggered by a variety of factors, but the most important are exposure to sunlight, age-related hormonal changes or inflammatory skin conditions.

Hyperpigmentation typically appears on areas of the skin that are regularly exposed to the sun, such as the hands or face. Ultraviolet radiation (UVA) contributes to melanogenesis by producing cellular oxidative stress, neutralising antioxidant defences.

Abnormal skin pigmentation is a significant aesthetic condition that can affect a person's quality of life. In addition, an overproduction of melanin can be associated with melanoma, a type of skin cancer. Therefore, it is important to study and develop products capable of effectively regulating melanin overproduction, such as composition A, whose effects on melanocytes are illustrated in Example 4.

**EXAMPLE 4. Composition A according to the present invention exhibits bleaching effects by inhibiting melanin synthesis and cell damage induced by exposure to ultraviolet radiation.**

Methodology

Normal human epidermal melanocytes were cultured for 72 hours together with composition A according to the present invention. During the incubation period, the cells were exposed to ultraviolet radiation twice daily (total dose = 5 $J/cm^2$). After the last radiation, the samples were incubated for an additional 96 hours prior to quantifying melanin and cell viability.

Results

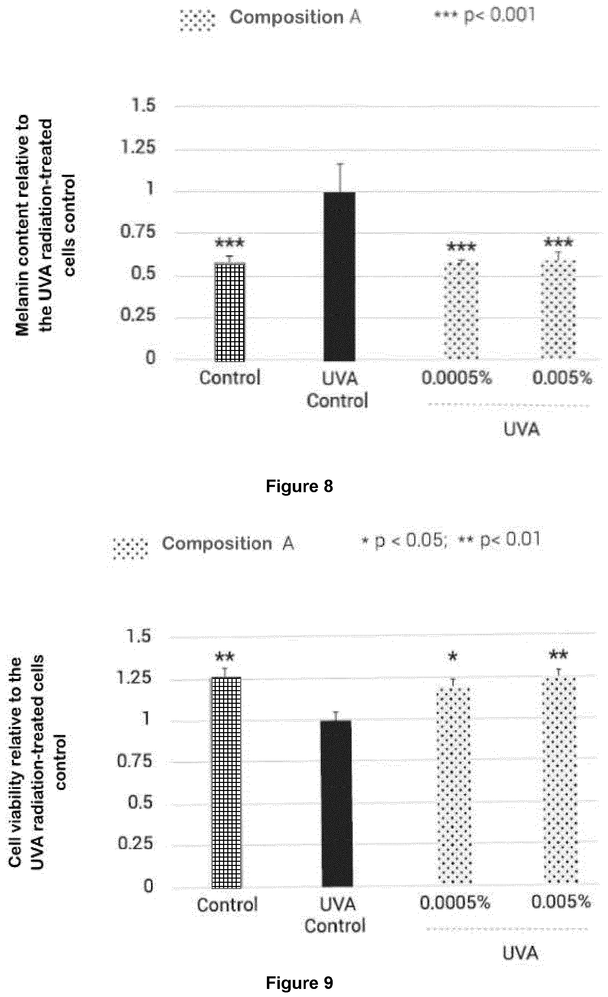

The results obtained can be seen in the graphs of Figure 8, for melanin production, and Figure 9, for cell viability.

Ultraviolet radiation (UVA) significantly increased melanin levels by 41%, compared to the control without UVA exposure. Composition A according to the invention at concentrations of 0.005% and 0.0005% by weight significantly reduced melanin levels by 41 and 40%, respectively, compared to untreated irradiated cells.

Ultraviolet radiation significantly reduced human melanocyte viability by 25% compared to the non-irradiated control. Composition A according to the invention at concentrations of 0.005% and 0.0005% by weight significantly increased cell survival and reduced cell damage caused by UVA radiation by 25% and 20%, respectively, compared to untreated irradiated cells.

**EXAMPLE 5. Composition A according to the present invention exhibits multiple highly significant beneficial effects on the skin as demonstrated in this clinical study in placebo-controlled humans.**

We selected 60 volunteers who showed symptoms of skin ageing. The volunteers were divided into 2 groups, one of them was administered a 225 mg daily dose of composition A and the other a dose of placebo (i.e. a capsule without composition A). The study lasted three months and measurements of the study parameters were made after 28 and 56 days and at the end of the study, after 84 days.

**Clinical Study Results**

Overall, composition A positively influences all monitored clinical parameters, both compared to the start of the study (T0), and compared to the placebo group from the first month of treatment (28 days), except for parameter R9, where the results obtained are statistically significant compared to the initial value, but are not significant compared to the group treated with placebo up to 56 days.

The table below shows the variation of each of the parameters versus the start of the study after 28, 56 and 84 days of treatment.

Performance of the study: the clinical study was conducted in parallel, randomised, double blinded groups in 60 volunteers with evident signs of ageing. The study lasted 12 weeks and the product efficacy was evaluated after 4, 8, and 12 weeks of taking the product. The parameters that were determined were: skin hydration (corneometer ®), transepidermal water loss (tewameter ®), skin luminosity and tone (spectrophotometer), skin elasticity and firmness (cutometer ®), wrinkle depth and skin roughness (3D Cousins ® image analysis), and epidermis and dermis thickness (ultrasound).

| | Composition A | | | Placebo | | |
|---|---|---|---|---|---|---|
| PARAMETERS | 28 d | 56 d | 84 d | 28 d | 56 d | 84 d |
| % variation vs. start (T0) | | | | | | |

(continued)

| PARAMETERS | Composition A | | | Placebo | | |
|---|---|---|---|---|---|---|
| | 28 d | 56 d | 84 d | 28 d | 56 d | 84 d |
| • Skin hydration | +7.1%$^{S*}$ | +15.3%$^{S*}$ | +19.6%$^{S*}$ | +3.7%$^{S}$ | +3.3%$^{nS}$ | +2.4%$^{nS}$ |
| • Transepidermal water loss | -5.7%$^{S*}$ | -10.6%$^{S*}$ | -14.3%$^{S*}$ | -1.8%$^{S}$ | -1.0%$^{nS}$ | -1.1%$^{nS}$ |
| • Wrinkle depth | -10.2%$^{S*}$ | -11.2%$^{S*}$ | -14.4%$^{S*}$ | +3.1%$^{S}$ | -2.2%$^{nS}$ | -3.7%$^{S}$ |
| • Smoothness parameter Ra [1] | -2.3%$^{S*}$ | -2.9%$^{S*}$ | -5.3%$^{S*}$ | +1.3%$^{NS}$ | +0.8%$^{NS}$ | -0.1%$^{NS}$ |
| • Overall skin elasticity - Parameter R2 | +6.8%$^{S*}$ | +11.8%$^{S*}$ | +14.2%$^{S*}$ | -0.8%$^{nS}$ | +0.4%$^{nS}$ | +1.7%$^{nS}$ |
| • Skin firmness - Parameter R0 [2] | -5.5%$^{S*}$ | -9.9%$^{S*}$ | -11.7%$^{S*}$ | -1.3%$^{nS}$ | -1.1%$^{nS}$ | -1.9%$^{nS}$ |
| • Net elasticity - Parameter R5 [3] | +5.8%$^{3*}$ | +14.1%$^{S*}$ | +17.3%$^{S*}$ | -1.2%$^{nS}$ | +1.1%$^{nS}$ | +1.5%$^{nS}$ |
| • Skin tiredness effects - R9 [4] | -5.1%$^{S}$ | -9.6%$^{S*}$ | -11.7%$^{S*}$ | -2.0%$^{nS}$ | -4.0%$^{S}$ | -4.7%$^{S}$ |
| • Skin colour (ITA Angle) [5] | +19.6%$^{S*}$ | +23.5%$^{S*}$ | +26.2%$^{S*}$ | +11.1%$^{S}$ | +9.2%$^{S}$ | +13.4%$^{S}$ |
| • Skin brightness (brightness parameter) | +13.9%$^{S*}$ | +23.7%$^{S*}$ | +27.3%$^{S*}$ | +7.2%$^{S}$ | +7.8%$^{S}$ | +9.5%$^{S}$ |
| variation vs. start (T0) | | | | | | |
| • Skin thickness (mm) | +0.06$^{S*}$ | +0.11%$^{S*}$ | +0.15%$^{S*}$ | +0.02$^{S}$ | +0.03$^{S}$ | +0.05$^{S}$ |

(1) A decrease in the parameter Ra can be expressed in absolute values as an increase in skin smoothness.
(2) A decrease in the parameter R0 can be expressed in absolute values as an increase in skin firmness.
(3) An improvement of the parameter R5 indicates an improvement of the elastic recovery of the skin after its deformation.
(4) The closer this value is to 0, the lower the tiredness effects are. Its decrease indicates an improvement in the skin.
(5) A low ITA angle indicates a brown pigmentation, while a high ITA angle value indicates clear pigmentation.
S: statistically significant vs. start of study (T0)($p<0.05$)|
*: statistically significant vs. placebo ($p<0.05$)|
NS: not statistically significant vs. start of study (T0)($p>0.05$)

In view of these results, it can be concluded that composition A exerts an exceptional and very significant benefit on all the skin's properties such as improving hydration and elasticity, reducing wrinkles and favouring the redensification of the dermis and epidermis, as a result of the synergistic effect of all its components on the telomeres of the epithelial cells. Furthermore, it is able to reduce the skin's dark spots and provide greater luminosity by making the skin acquire a more uniform tone.

**Claims**

1. A composition of plant extracts to mitigate the effects of ageing, **characterised in that** it comprises an asiaticoside content of at least 6% by weight, a verbascoside content of at least 2.5% by weight, a hesperidin content of at least 12.5% by weight and a punicalagin content of at least 3% by weight.

2. A composition according to claim 1, **characterised in that** the asiaticoside content is provided as an centella asiatica extract.

3. A composition according to claim 2, **characterised in that** the composition contains about 36% by weight of centella asiatica extract.

4. A composition according to any preceding claim, **characterised in that** the verbascoside content is provided as a cistanche extract.

5. A composition according to claim 4, **characterised in that** the composition contains about 32% by weight of cistanche extract.

6. A composition according to claim 4 or 5, **characterised in that** the cistanche extract is of the following cistanche varieties: *Cistanche tubulosa, Cistanche salsa* and/or *Cistanche deserticola.*

7. A composition according to any preceding claim, **characterised in that** the verbascoside content is provided with plant extracts such as *Lippia citriodora, Budeja sp., Rehmania glutinosa, Plantago spp.*

8. A composition according to any preceding claim, **characterised in that** the hesperidin content is provided as a citrus extract.

9. A composition according to claim 8, **characterised in that** the composition contains about 23% sweet orange extract.

10. A composition according to any preceding claim, **characterised in that** the punicalagin content is provided as a pomegranate extract (*Punica granatum*).

11. A composition according to claim 10, **characterised in that** it contains about 9% pomegranate extract.

12. A composition according to any preceding claim, **characterised in that** it further reduces melanin production in skin exposed to solar radiation.

13. A formulation to mitigate the effects of ageing, **characterised in that** it comprises a composition according to any one of claims 1 to 12 and is further configured to be administered orally containing one or more physiologically compatible carriers or excipients, in solid or liquid form.

14. A formulation to mitigate the effects of ageing, **characterised in that** it comprises a composition according to any one of claims 1 to 12 and is further configured to be administered topically containing one or more carriers or excipients physiologically compatible with human skin.

Figure 1

**Figure 2**

Figure 3

**Figure 4**

Legend: Control; Composition A 0.001%; Composition A 0.0001%; Composition A 0.0005%; Composition A 0.0001%

Y-axis: Relative Telomerase Activity (%)

X-axis: Time (hours)

**Figure 5**

**Figure 6**

Figure 7

Figure 8

Figure 9

| INTERNATIONAL SEARCH REPORT | International application No. PCT/ES2023/070157 |
|---|---|

**A. CLASSIFICATION OF SUBJECT MATTER**

*A61K8/97* (2017.01)
*A61Q19/08* (2006.01)
According to International Patent Classification (IPC) or to both national classification and IPC

**B. FIELDS SEARCHED**

Minimum documentation searched (classification system followed by classification symbols)
A61K, A61Q

Documentation searched other than minimum documentation to the extent that such documents are included in the fields searched

Electronic data base consulted during the international search (name of data base and, where practicable, search terms used)

INVENES, EPODOC, WPI, BIOSIS, EMBASE, MEDLINE, INTERNET, XPESP, NPL y bases de texto TXT.

**C. DOCUMENTS CONSIDERED TO BE RELEVANT**

| Category* | Citation of document, with indication, where appropriate, of the relevant passages | Relevant to claim No. |
|---|---|---|
| A | CN 105287310 A (JIANGSU LONGRICH BIOTECH CO LTD) 03/02/2016, claims, abstract | 1-14 |
| A | WO 2004069218 A1 (LABORATOIRES DE BIOLOGIE VEGETALE YVES ROCHER) 03/02/2004, Claims and abstract | 1-14 |
| A | CN 102038702 A (UNIV ZHEJIANG) 02/11/2010, claims and abstract | 1-14 |
| A | US 2006002884 A1 (COTY BV [NL]) 23/06/2003, claims, paragraph [0016] | 1-14 |

☒ Further documents are listed in the continuation of Box C.     ☒ See patent family annex.

| * Special categories of cited documents: | "T" | later document published after the international filing date or priority date and not in conflict with the application but cited to understand the principle or theory underlying the invention |
|---|---|---|
| "A" document defining the general state of the art which is not considered to be of particular relevance. | | |
| "E" earlier document but published on or after the international filing date | | |
| "L" document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" document published prior to the international filing date but later than the priority date claimed | "&" | document member of the same patent family |

| Date of the actual completion of the international search 02/06/2023 | Date of mailing of the international search report **(06/06/2023)** |
|---|---|
| Name and mailing address of the ISA/ OFICINA ESPAÑOLA DE PATENTES Y MARCAS Paseo de la Castellana, 75 - 28071 Madrid (España) Facsimile No.: 91 349 53 04 | Authorized officer A. Ugidos Valladares Telephone No. 91 3495567 |

Form PCT/ISA/210 (second sheet) (July 2022)

## INTERNATIONAL SEARCH REPORT

| International application No. |
| --- |
| PCT/ES2023/070157 |

C (continuation).             DOCUMENTS CONSIDERED TO BE RELEVANT

| Category * | Citation of documents, with indication, where appropriate, of the relevant passages | Relevant to claim No. |
| --- | --- | --- |
| A | HALDER S. et al. Herbal drugs and natural bioactive products as potential therapeutics: A review on pro-cognitives and brain boosters perspectives. Saudi Pharm J. 2021 Aug; 29(8): 879–907., 15/07/2021 [on line][retrieved on 31/05/2023 ]. <DOI: 10.1016/j.jsps.2021.07.003> page 882,column 2; tabl2 2 compound 49; table 3, compound 17; table 4, compound number 1 | 1-14 |

Form PCT/ISA/210 (continuation of second sheet) (July 2022)

## INTERNATIONAL SEARCH REPORT

Information on patent family members

International application No.

PCT/ES2023/070157

| Patent document cited in the search report | Publication date | Patent family member(s) | Publication date |
|---|---|---|---|
| CN105287310 A | 03.02.2016 | NONE | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Targeting Senescent Cells for a Healthier Aging: Challenges and Opportunities. **SHULING SONG** ; **TAMARA TCHKONIA** ; **JING JIANG** ; **JAMES L. KIRKLAND** ; **YU SUN**. Advanced Science. Wiley, December 2020, vol. 7
- **BUCKINGHAM EM** ; **KLINGELHUTZ AJ**. The role of telomeres in the ageing of human skin. *Exp Dermatol.*, April 2011, vol. 20 (4), 297-302
- **D'ERRICO M** ; **LEMMA T** ; **CALCAGNILE A** ; **PROIETTI DE SANTIS L** ; **DOGLIOTTI E**. Cell type and DNA damage specific response of human skin cells to environmental agents. *Mutat Res.*, 03 January 2007, vol. 614 (1-2), 37-47
- **MIYATA Y** ; **OKADA K** ; **FUJIMOTO A** ; **HATA K** ; **KAGAMI H** ; **TOMITA Y** ; **UEDA M**. The effect of long-term cultivation on telomere length and morphology of cultured epidermis. *J Dermatol Sci.*, May 2004, vol. 34 (3), 221-30
- **MOYZIS RK** ; **BUCKINGHAM JM** ; **CRAM LS** ; **DANI M** ; **DEAVEN LL** ; **JONES MD** ; **MEYNE J** ; **RATLIFF RL** ; **WU JR**. A highly conserved repetitive DNA sequence, (TTAGGG)n, present at the telomeres of human chromosomes. *Proc Natl Acad Sci U S A.*, September 1988, vol. 85 (18), 6622-6
- **O'SULLIVAN RJ** ; **KARLSEDER J**. Telomeres: protecting chromosomes against genome instability. *Nat Rev Mol Cell Biol.*, March 2010, vol. 11 (3), 171-81

- **SMEETS SJ** ; **VAN DER PLAS M** ; **SCHAAIJ-VISSER TB** ; **VAN VEEN EA** ; **VAN MEERLOO J** ; **BRAAKHUIS BJ** ; **STEENBERGEN RD** ; **BRAKENHOFF RH**. Immortalization of oral keratinocytes by functional inactivation of the p53 and pRb pathways. *Int J Cancer.*, 01 April 2011, vol. 128 (7), 1596-605
- **SANDRA FREITAS-RODRÍGUEZ** ; **ALICIA R. FOLGUERAS** ; **CARLOS LÓPEZ-OTÍN**. The role of matrix metalloproteinases in aging: Tissue remodeling and beyond. *Biochimica et Biophysica Acta (BBA) - Molecular Cell Research*, 2007, vol. 1864 (11), 2015-2025
- **CHAN SR** ; **BLACKBURN EH**. Telomeres and telomerase. *Philos Trans R Soc Lond B Biol Sci.*, 2004, vol. 359 (1441), 109-121
- **VICTORELLI S** ; **PASSOS JF**. Telomeres and cell senescence - size matters not. *EBioMedicine*, 2017, vol. 21, 14-20
- **EISENBERG DTA**. An evolutionary review of human telomere biology: the thrifty telomere hypothesis and notes on potential adaptive paternal effects. *American Journal of Human Biology*, 2011, vol. 23, 149-167
- **OESEBURG H et al.** Telomere biology in healthy aging and disease. *Pflügers Archiv - European Journal of Physiology*, 2010, vol. 459, 259-268
- **SAHIN E** ; **DEPINHO RA**. Linking functional decline of telomeres, mitochondria and stem cells during ageing. *Nature*, 2010, vol. 464, 520-528
- **HOU M** ; **XU D** ; **BJORKHOLM M** ; **GRUBER A**. Real-time quantitative telomeric repeat amplification protocol assay for the detection of telomerase activity. *Clin Chem.*, 2001, vol. 47 (3), 519-524